# EUROPEAN PATENT APPLICATION

(11) **EP 4 268 792 A1**
(43) Date of publication of application: **01.11.2023**
(21) Application number: 21915324.4
(22) Date of filing: 28.12.2021
(51) Int. Cl.: A61K 6/60, A61K 6/35, A61K 6/62, A61L 24/06

(54) **ADHESIVE COMPOSITION FOR PHOTOFABRICATION ARTICLES**

(30) Priority: 28.12.2020 JP 2020219727
(71) Applicant: Kuraray Noritake Dental Inc., Kurashiki-shi, Okayama 710-0801 (JP)
(72) Inventor: SUZUKI, Kenji, Tainai-shi, Niigata 959-2653 (JP)
(74) Representative: D Young & Co LLP
(86) International application number: PCT/JP2021/048912
(87) International publication number: WO 2022/145466

(57) **Abstract**

The present invention provides a stereolithographic article adhesive composition having excellent adhesiveness with respect to a shaped article produced by stereolithography. The present invention relates to a stereolithographic article adhesive composition comprising a multifunctional (meth)acrylic-based polymerizable monomer (A) and a photopolymerization initiator (B). The multifunctional (meth)acrylic-based polymerizable monomer (A) preferably contains at least one of a urethanated (meth)acrylic acid ester compound (a-1), an aromatic compound-based (meth)acrylic acid ester compound (a-2) containing no urethane bond, and a (meth)acrylamide group-containing polymerizable monomer (a-3).

## Description

### TECHNICAL FIELD

The present invention relates to a stereolithographic article adhesive composition. More specifically, the present invention relates to a stereolithographic article adhesive composition having excellent adhesiveness with respect to a shaped article produced by stereolithography, and the stereolithographic article adhesive composition is capable of restoring or lining a shaped article produced by stereolithography. In particular, the stereolithographic article adhesive composition is suitable for lining or restoring a denture base material, a dental occlusal splint, and a sleep disorder treatment tool.

### BACKGROUND ART

Numerous proposals have been made concerning a so-called optical three-dimensional shaping method in which a three-dimensionally shaped article is produced by repeating a process of supplying a necessary amount of controlled light energy to a liquid photocurable resin to cure the liquid photocurable resin in a thin layer shape, further supplying a liquid photocurable resin on top of the cured resin, and then irradiating the liquid photocurable resin with light under control to cure the liquid photocurable resin in a thin layer shape in a laminated manner.

As a typical method for optically producing a three-dimensionally shaped article, the following VAT stereolithography is generally employed. The liquid surface of a liquid photocurable resin composition in a container is selectively irradiated with a computer-controlled ultraviolet laser so as to obtain a desired pattern to cure the resin composition at a predetermined thickness to form a cured layer. Next, a liquid photocurable resin composition for one layer is supplied on top of the cured layer, and is irradiated with an ultraviolet laser in the same manner to cure the resin composition in the same manner as described above to form a continuous cured layer. Such lamination operations are repeated to produce a three-dimensionally shaped article having a final shape. This method has been attracting a great deal of attention in recent years since it allows a desired three-dimensionally shaped article to be easily produced with high accuracy in a relatively short time even if the shape of the three-dimensionally shaped article is quite complicated.

The applications of three-dimensionally shaped articles obtained by stereolithography are expanding from mere concept models to test models, prototypes, and final products. In particular, in the field of dental materials, denture bases and mouthpiece-like sleep disorder treatment tools such as tools for preventing teeth grinding and treatment tools for sleep apnea syndrome are different in shape for each individual patient, and have complicated shapes. Therefore, practical use of stereolithography therefor is expected.

A denture base material is a material used for a gingival portion when a denture is attached due to tooth loss. In recent years, the demand for dentures has increased dramatically with the increase in the elderly population. A dentureenture base material is frequently lined (sometimes called rebased or relined) or restored due to deformation of the material itself, changes in the patient's crest, etc. For lining and restoring a denture base, a method is used in which the surface thereof is treated as necessary with an adhesive material obtained by dissolving a methacrylic acid ester-based polymer in an organic solvent such as ethyl acetate or methylene chloride and then a mixture of a methacrylic acid ester-based monomer and a methacrylic acid ester-based polymer, containing methyl methacrylate as a main agent, is applied thereto. This is the only method in actual use. Mouthpiece-like sleep disorder treatment tools such as tools for preventing teeth grinding and treatment tools for sleep apnea syndrome are also restored in a similar manner.

As for the production of a denture base itself, the conventional mainstream method is to produce a denture base material from a mixture of methyl methacrylate (MMA) and polymethyl methacrylate (PMMA) by thermal polymerization. Meanwhile, a denture base material can also be produced by stereolithography. In this case, in stereolithography, the denture base material is instantly cured by light, and thus, for the denture base material, it is necessary to use a polymerizable compound having particularly high curability, and a crosslinkable monomer or a monomer that exhibits high cohesion is used. In addition, due to the structure of a stereolithography apparatus, volatile components cannot be used, and thus the use of MMA is not practical. For these reasons, a denture base material produced by stereolithography has a high crosslink density and it is difficult for a denture base lining material or a denture base lining adhesive material to permeate the denture base material, so that there is a problem that lining and restoration are difficult with the denture base material. In addition, for the conventional lining and restoration, a solvent such as methylene chloride and ethyl acetate is used for the adhesive material, and MMA is used for a lining material, so that there is also a problem of significant odor.

As dental adhesive materials (adhesive compositions), those applied to dentins, those applied to dental composite resins, and those applied to metal or ceramic dental prostheses are widely known. Dental adhesive materials for these applications are materials, such as acidic monomers, silane coupling agents, and sulfur-containing monomers, which have excellent affinity to each adherend such as dentins and which form chemical bonds. However, adhesive materials for denture base materials, dental occlusal splints, and sleep disorder treatment tools are completely different from the adhesive materials for these applications in technology and mechanism for exhibiting adhesiveness.

Against this background, as a nonconventional denture base lining method, for example, Patent Literature 1 describes an example of a composition with excellent adhesiveness obtained by diluting a mixture of monofunctional and multifunctional (meth)acrylic-based monomers in a solvent. Also, Patent Literature 2 describes an example of a photocurable lining material having excellent curability. Moreover, Patent Literature 3 describes an example of a denture base material with excellent strength and toughness for stereolithography.

### CITATION LIST

### Patent Literature

Patent Literature 1: JP 2008-214359 A
Patent Literature 2: JP 2019-44065 A
Patent Literature 3: WO 2018/181832

### SUMMARY OF INVENTION

### Technical Problem

However, in Patent Literatures 1 and 2, PMMA-based denture base materials are targeted for lining, and there is no mention of targeting highly-crosslinked stereolithographic articles. In addition, Patent Literature 3 discloses a denture base itself, and does not describe the use of a denture base lining material and a denture base lining adhesive material.

Therefore, the present invention aims to provide a stereolithographic article adhesive composition having excellent adhesiveness with respect to a shaped article produced by stereolithography. In particular, the stereolithographic article adhesive composition is suitable for lining a denture base material, a dental occlusal splint, and a sleep disorder treatment tool and for a lining adhesive material.

### Solution to Problem

That is, the present invention includes the following invention.
[1] A stereolithographic article adhesive composition comprising a multifunctional (meth)acrylic-based polymerizable monomer (A) and a photopolymerization initiator (B).
[2] The stereolithographic article adhesive composition according to [1], wherein the multifunctional (meth)acrylic-based polymerizable monomer (A) contains at least one of a urethanated (meth)acrylic acid ester compound (a-1), an aromatic compound-based (meth)acrylic acid ester compound (a-2) containing no urethane bond, and a (meth)acrylamide group-containing polymerizable monomer (a-3).
[3] The stereolithographic article adhesive composition according to [2], wherein the aromatic compound-based (meth)acrylic acid ester compound (a-2) containing no urethane bond has at least one group selected from the group consisting of a hydroxyl group, a primary amino group, and a secondary amino group.
[4] The stereolithographic article adhesive composition according to [2], wherein the (meth)acrylamide group-containing polymerizable monomer (a-3) contains at least one bond selected from the group consisting of a primary amide bond and a secondary amide bond.
[5] The stereolithographic article adhesive composition according to [2], wherein the urethanated (meth)acrylic acid ester compound (a-1) is a compound containing no polymer structure.
[6] The stereolithographic article adhesive composition according to [5], wherein the urethanated (meth)acrylic acid ester compound (a-1) contains at least one selected from the group consisting of 2,2,4-trimethylhexamethylenebis(2-carbamoyloxyethyl) dimethacrylate and N,N'-(2,2,4-trimethylhexamethylene)bis[2-(aminocarboxy)propane-1 ,3-diol] tetramethacrylate.
[7] The stereolithographic article adhesive composition according to any one of [1] to [6], further comprising a monofunctional (meth)acrylic-based polymerizable monomer (C).
[8] The stereolithographic article adhesive composition according to [7], wherein the monofunctional (meth)acrylic-based polymerizable monomer (C) has an atmospheric-equivalent boiling point of 250°C or higher.
[9] The stereolithographic article adhesive composition according to [7] or [8], wherein the monofunctional (meth)acrylic-based polymerizable monomer (C) contains no urethane bond, no hydroxyl group, no amino group, and no acidic group.
[10] The stereolithographic article adhesive composition according to any one of [7] to [9], wherein the monofunctional (meth)acrylic-based polymerizable monomer (C) contains an aromatic ring.
[11] The stereolithographic article adhesive composition according to any one of [1] to [10], wherein the stereolithographic article is a denture base material, a dental mouthpiece, or a treatment tool for sleep apnea syndrome.
[12] A denture base lining material comprising the stereolithographic article adhesive composition according to any one of [1] to [10].
[13] A denture base lining adhesive material comprising the stereolithographic article adhesive composition according to any one of [1] to [10].
[14] A sleep disorder treatment tool restoring material comprising a stereolithographic article of the stereolithographic article adhesive composition according to any one of [1] to [10].
[15] A method for lining or restoring a three-dimensionally shaped article produced by an optical three-dimensional shaping method, using the stereolithographic article adhesive composition according to any one of [1] to [11].

### Advantageous Effects of Invention

According to the present invention, it is possible to provide a stereolithographic article adhesive composition having excellent adhesiveness with respect to a shaped article produced by stereolithography (hereinafter, also referred to as "stereolithographic article"). In addition, the stereolithographic article adhesive composition of the present invention easily permeates a stereolithographic article having a high crosslink density, and thus is capable of restoring or lining a stereolithographic article. In particular, the stereolithographic article adhesive composition of the present invention can be suitably used for lining a denture base material, a dental occlusal splint, and a sleep disorder treatment tool. Furthermore, the stereolithographic article adhesive composition of the present invention has a low odor and is less likely to produce unpleasant odors.

### DESCRIPTION OF EMBODIMENTS

The stereolithographic article adhesive composition of the present invention comprises a multifunctional (meth)acrylic-based polymerizable monomer (A) and a photopolymerization initiator (B). In the present specification, the upper limits and lower limits of numeric ranges (contents of components, values and physical properties calculated from components, etc.) can be combined as appropriate. In the present specification, the numeric values of symbols in formulae can also be combined as appropriate.

### [Multifunctional (meth)acrylic-based polymerizable monomer (A)]

The multifunctional (meth)acrylic-based polymerizable monomer (A) is used in the stereolithographic article adhesive composition of the present invention to impart excellent adhesiveness with respect to a stereolithographic article. Since curability particularly strongly contributes to exhibiting excellent adhesiveness, the multifunctional (meth)acrylic-based polymerizable monomer (A) needs to contain two or more (meth)acrylic-based polymerizable groups ((meth)acryloyloxy group, (meth)acrylamide group) in one molecule. From the viewpoint of exhibiting particularly high curability and easily achieving excellent adhesiveness with respect to a stereolithographic article, at least one of a urethanated (meth)acrylic acid ester compound (a-1), an aromatic compound-based (meth)acrylic acid ester compound (a-2) containing no urethane bond, and a (meth)acrylamide group-containing polymerizable monomer (a-3) is preferably contained. As the multifunctional (meth)acrylic-based polymerizable monomer (A), one monomer may be used alone, or two or more monomers may be used in combination. Among them, from the viewpoint of easily achieving excellent adhesiveness with respect to a stereolithographic article, the aromatic compound-based (meth)acrylic acid ester compound (a-2) containing no urethane bond more preferably contains at least one group selected from the group consisting of a hydroxyl group, a primary amino group, and a secondary amino group. From the viewpoint of easily achieving excellent adhesiveness with respect to a stereolithographic article, the (meth)acrylamide group-containing polymerizable monomer (a-3) more preferably contains at least one bond selected from the group consisting of a primary amide bond and a secondary amide bond. These compounds are inferred to not only promote curing but also form hydrogen bonds and chemical bonds due to π-electron action through the dielectric effects of urethane bonds, aromatic skeletons, acrylamides, hydroxyl groups, primary amino groups, and secondary amino groups. Therefore, the adhesiveness with respect to a shaped article is excellent. In the present specification, the term "(meth)acrylic" is used to mean both methacrylic and acrylic, and the same applies to terms "(meth)acryloyl", "(meth)acrylate", etc., which are similar thereto. The term "(meth)acrylate-based" is used to mean both (meth)acrylic acid ester and (meth)acrylamide.

### [Urethanated (meth)acrylic acid ester compound (a-1)]

The urethanated (meth)acrylic acid ester compound (a-1) can be easily synthesized, for example, by an addition reaction of a compound containing an isocyanate containing an alkylene skeleton or a phenylene skeleton with a (meth)acrylate compound having a hydroxyl group. Meanwhile, from the viewpoint of low viscosity, easy application, and easily obtaining permeability into a stereolithographic article, the weight average molecular weight of the urethanated (meth)acrylic acid ester compound (a-1) is preferably 1,000 or less. In the case where the urethanated (meth)acrylic acid ester compound (a-1) contains no polymer structure, there is no concept of weight average molecular weight, and thus molecular weight is used. When the weight average molecular weight exceeds 1,000, the stereolithographic article adhesive composition tends to have a higher viscosity and lower permeability. From the viewpoint of excellent adhesiveness with respect to a stereolithographic article, the weight average molecular weight is more preferably 750 or less and further preferably 500 or less. Furthermore, the urethanated (meth)acrylic acid ester compound (a-1) is preferably a compound containing no polymer structure of a polyester, a polycarbonate, a polyurethane, a polyether, or the like. When these structures are contained, the concentration of functional groups having polarity increases, and thus the adhesiveness tends to decrease in the presence of moisture, such as in an oral cavity. In particular, from the viewpoint of adhesiveness in the presence of moisture, the number of urethane bonds in one molecule is more preferably three or less and further preferably two or less. As used herein, the weight average molecular weight means a weight average molecular weight determined by gel permeation chromatography (GPC) in terms of polystyrene. As the urethanated (meth)acrylic acid ester compound (a-1), one compound may be used alone, or two or more compounds may be used in combination.

Examples of the compound having an isocyanate group include methylene diisocyanate (MDI), hexamethylene diisocyanate (HDI), isophorone diisocyanate (IPDI), trimethylhexamethylene diisocyanate (TMHMDI), tricyclodecane diisocyanate (TCDDI), adamantane diisocyanate (ADI), tolylene diisocyanate (TDI), xylylene diisocyanate (XDI), and diphenylmethane diisocyanate (MDI). Among them, from the viewpoint that the stereolithographic article adhesive composition of the present invention has excellent adhesiveness with respect to a stereolithographic article, HDI, IPDI, TMHMDI, and TCDDI are preferable, and IPDI and TMHMDI are more preferable.

Examples of the (meth)acrylate compound having a hydroxyl group include hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2 hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-[3-(meth)acryloyloxy-2-hydroxypropoxy]phenyl]propane, 1,2-bis[3-(meth)acryloyloxy-2-hydroxypropoxy]ethane, pentaerythritol tri(meth)acrylate, and dipentaerythritol tri or tetra(meth)acrylate. One of these compounds may be used alone, or two or more of these compounds may be used in combination. Among them, from the viewpoint that the stereolithographic article adhesive composition of the present invention has excellent adhesiveness with respect to a stereolithographic article, 2-hydroxyethyl (meth)acrylate, 2-hydroxypropyl (meth)acrylate, and 2-hydroxy-3-acryloyloxypropyl (meth)acrylate are preferable, and 2-hydroxyethyl (meth)acrylate is more preferable.

The addition reaction of the compound having an isocyanate group with the (meth)acrylate compound having a hydroxyl group can be carried out according to a known method, and is not particularly limited.

Examples of the urethanated (meth)acrylic acid ester compound containing no polymer structure include 2,2,4-trimethylhexamethylenebis(2-carbamoyloxyethyl) dimethacrylate (commonly known as UDMA), 2,4-tolylenebis(2-carbamoyloxyethyl) dimethacrylate, bishydroxyethyl methacrylate-isophorone diurethane, 2,4-tolylenebis(2-carbamoyloxyethyl) dimethacrylate, N,N'-(2,2,4-trimethylhexamethylene)bis[2-(aminocarboxy)propane-1 ,3-diol] tetramethacrylate, hexamethylenebis{2-carbamoyloxy-3-phenoxypropyl} diacrylate, and 2,4-tolylenebis(2-carbamoyloxyethyl) hexaacrylate. One of these compounds may be used alone, or two or more of these compounds may be used in combination. Among them, from the viewpoint of adhesiveness with respect to a stereolithographic article, 2,2,4-trimethylhexamethylenebis(2-carbamoyloxyethyl) dimethacrylate and N,N'-(2,2,4-trimethylhexamethylene)bis[2-(aminocarboxy)propane-1 ,3-diol] tetramethacrylate are preferable, and 2,2,4-trimethylhexamethylenebis(2-carbamoyloxyethyl) dimethacrylate is more preferable.

In the case where the stereolithographic article adhesive composition of the present invention contains the urethanated (meth)acrylic acid ester compound (a-1), in an embodiment that does not contain a monofunctional (meth)acrylic-based polymerizable monomer (C), the content of the urethanated (meth)acrylic acid ester compound (a-1) in the stereolithographic article adhesive composition of the present invention is preferably 10 to 100 parts by mass in 100 parts by mass of the total amount of the polymerizable monomer. From the viewpoint of better adhesiveness with respect to a stereolithographic article, the content of the urethanated (meth)acrylic acid ester compound (a-1) is more preferably 30 to 100 parts by mass and further preferably 50 to 100 parts by mass. In an embodiment that includes the monofunctional (meth)acrylic-based polymerizable monomer (C), the content of the urethanated (meth)acrylic acid ester compound (a-1) is preferably 10 to 98 parts by mass, more preferably 30 to 90 parts by mass, and further preferably 50 to 80 parts by mass in 100 parts by mass of the total amount of the polymerizable monomer.

In a certain embodiment (e.g., an embodiment that does not contain the monofunctional (meth)acrylic-based polymerizable monomer (C)), the content of the urethanated (meth)acrylic acid ester compound (a-1) in the stereolithographic article adhesive composition is preferably 40 to 99.9 mass% in the total amount of the stereolithographic article adhesive composition. From the viewpoint of better adhesiveness with respect to a stereolithographic article, the content of the urethanated (meth)acrylic acid ester compound (a-1) is more preferably 55 to 99.5 mass% and further preferably 60 to 99 mass%. In another certain embodiment (e.g., an embodiment that includes the monofunctional (meth)acrylic-based polymerizable monomer (C)), the content of the urethanated (meth)acrylic acid ester compound (a-1) in the stereolithographic article adhesive composition is preferably 40 to 98 mass% in the total amount of the stereolithographic article adhesive composition. From the viewpoint of better adhesiveness with respect to a stereolithographic article, the content of the urethanated (meth)acrylic acid ester compound (a-1) is more preferably 45 to 90 mass% and further preferably 55 to 85 mass%.

### [Aromatic compound-based (meth)acrylic acid ester compound (a-2) containing no urethane bond]

Examples of the aromatic compound-based (meth)acrylic acid ester compound (a-2) containing no urethane bond include 2,2-bis((meth)acryloyloxyphenyl)propane, 2,2-bis[4-(3-acryloyloxy)-2hydroxypropoxyphenyl]propane, 2,2-bis[4-(3-methacryloyloxy-2-hydroxypropoxy)phenyl]propane (commonly known as "Bis-GMA"), 2,2-bis[4-{2-(3-acryloyloxy-2-hydroxypropoxy)propyl}phenyl]propane, 2,2-bis[4-{2-(3-methacryloyloxy-2-hydroxypropoxy)propyl}phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl)pyromellitate. One of these compounds may be used alone, or two or more of these compounds may be used in combination. Among them, from the viewpoint of excellent adhesiveness with respect to a stereolithographic article, a hydroxyl group is preferably contained, and specifically, 2,2-bis[4-(3-acryloyloxy)-2-hydroxypropoxyphenyl]propane, 2,2-bis[4-(3-methacryloyloxy-2-hydroxypropoxy)phenyl]propane, 2,2-bis[4-{2-(3-acryloyloxy-2-hydroxypropoxy)propyl}phenyl]propane, 2,2-bis[4-{2-(3-methacryloyloxy-2-hydroxypropoxy)propyl}phenyl]propane are preferable. Among them, 2,2-bis[4-(3-methacryloyloxy-2-hydroxypropoxy)phenyl]propane is more preferable.

In the case where the stereolithographic article adhesive composition of the present invention includes the aromatic compound-based (meth)acrylic acid ester compound (a-2) containing no urethane bond, the content of the aromatic compound-based (meth)acrylic acid ester compound (a-2) containing no urethane bond in the stereolithographic article adhesive composition of the present invention is preferably 5 to 90 parts by mass in 100 parts by mass of the total amount of the polymerizable monomer. From the viewpoint of better adhesiveness with respect to a stereolithographic article, the content of the aromatic compound-based (meth)acrylic acid ester compound (a-2) containing no urethane bond is more preferably 10 to 80 parts by mass and further preferably 20 to 70 parts by mass.

In a certain embodiment (e.g., an embodiment that does not contain the monofunctional (meth)acrylic-based polymerizable monomer (C)), the content of the aromatic compound-based (meth)acrylic acid ester compound (a-2) containing no urethane bond in the stereolithographic article adhesive composition is preferably 40 to 99.9 mass% in the total amount of the stereolithographic article adhesive composition. From the viewpoint of better adhesiveness with respect to a stereolithographic article, the content of the aromatic compound-based (meth)acrylic acid ester compound (a-2) containing no urethane bond is more preferably 55 to 99.5 mass% and further preferably 60 to 99 mass%. In another certain embodiment (e.g., an embodiment that includes the monofunctional (meth)acrylic-based polymerizable monomer (C)), the content of the aromatic compound-based (meth)acrylic acid ester compound (a-2) containing no urethane bond in the stereolithographic article adhesive composition is preferably 40 to 98 mass% in the total amount of the stereolithographic article adhesive composition. From the viewpoint of better adhesiveness with respect to a stereolithographic article, the content of the aromatic compound-based (meth)acrylic acid ester compound (a-2) containing no urethane bond is more preferably 45 to 90 mass% and further preferably 55 to 85 mass%.

### [(Meth)acrylamide group-containing polymerizable monomer (a-3)]

Examples of the (meth)acrylamide group-containing polymerizable monomer (a-3) include: (meth)acrylamide-based polymerizable monomers such as N,N'-ethylenebis(meth)acrylamide, N,N'-propylenebis(meth)acrylamide, N,N'-butylenebis(meth)acrylamide, and N,N'-hexamethylene bis(meth)acrylamide; and polymerizable monomers having one (meth)acrylamide group and one (meth)acryloyloxy group such as 2-(meth)acryloyloxyethyl(meth)acrylamide, 2-(meth)acryloyloxypropyl(meth)acrylamide, 2-(meth)acryloyloxybutyl(meth)acrylamide, and 2-(meth)acryloyloxyhexyl(meth)acrylamide. A polymerizable monomer having one (meth)acrylamide group and one (meth)acryloyloxy group may be an asymmetric acrylamide-methacrylic acid ester compound represented by the following general formula (1). As the (meth)acrylamide group-containing polymerizable monomer (a-3), one of these monomers may be used alone, or two or more of these monomers may be used in combination.

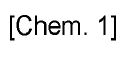

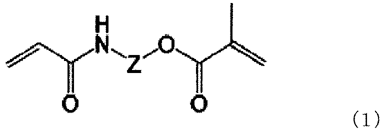

where Z is an optionally substituted C₁-C₈ linear or branched aliphatic or aromatic group, and the aliphatic group may be interrupted by at least one linking group selected from the group consisting of -O-, -S-, -CO-, -CO-O-, -O-CO-, -NR¹-, -CO-NR¹-, -NR¹-CO-, -CO-O-NR¹-, -O-CO-NR¹-, and -NR¹-CO-NR¹-. R¹ represents a hydrogen atom or an optionally substituted C₁-C₈ linear or branched aliphatic group.

Z is a moiety that adjusts the hydrophilicity of the asymmetric acrylamide methacrylic acid ester compound. The optionally substituted C₁-C₈ aliphatic group represented by Z may be either a saturated aliphatic group (alkylene group, cycloalkylene group (e.g., 1 ,4-cyclohexylene group, etc.)) or an unsaturated aliphatic group (alkenylene group, alkynylene group), and is preferably a saturated aliphatic group (alkylene group) from the viewpoint of ease of availability or production and chemical stability. From the viewpoint of adhesiveness with respect to dentins and polymerization curability, Z is preferably an optionally substituted linear or branched C₁-C₄ aliphatic group, and is more preferably an optionally substituted linear or branched C₂-C₄ aliphatic group. The aliphatic group is preferably an alkylene group. Examples of the C₁-C₈ alkylene group include a methylene group, an ethylene group, an n-propylene group, an isopropylene group, and an n-butylene group.

Examples of the optionally substituted aromatic group represented by Z include arylene groups and aromatic heterocyclic groups. As the aromatic group, arylene groups are more preferable than aromatic heterocyclic groups. The heterocyclic ring of an aromatic heterocyclic group is generally unsaturated. The aromatic heterocyclic ring is preferably a 5- or 6-membered ring. As the arylene groups, for example, phenylene groups are preferable. Examples of the hetero rings of the aromatic heterocyclic groups include a furan ring, a thiophene ring, a pyrrole ring, an oxazole ring, an isoxazole ring, a thiazole ring, an isothiazole ring, an imidazole ring, a pyrazole ring, a furazan ring, a triazole ring, a pyran ring, a pyridine ring, a pyridazine ring, a pyrimidine ring, a pyrazine ring, and a 1,3,5-triazine ring. Among the aromatic groups, phenylene groups are particularly preferable.

The aliphatic group in R¹ may be either a saturated aliphatic group (alkyl group) or an unsaturated aliphatic group (alkenyl group, alkynyl group), and is preferably a saturated aliphatic group (alkyl group) from the viewpoint of ease of availability or production and chemical stability. Examples of the alkyl group in R¹ include a C₁-C₆ linear or branched alkyl group, a C₁-C₃ linear alkyl group is preferable, a methyl group or an ethyl group is more preferable, and a methyl group is further preferable.

As the (meth)acrylamide group-containing polymerizable monomer (a-3), among them, from the viewpoint of excellent adhesiveness with respect to a stereolithographic article, 2-(meth)acryloyloxyethyl(meth)acrylamide is preferable, and 2-methacryloyloxyethylacrylamide is more preferable.

In an embodiment that does not contain the monofunctional (meth)acrylic-based polymerizable monomer (C), the content of the (meth)acrylamide group-containing polymerizable monomer (a-3) in the stereolithographic article adhesive composition of the present invention is preferably 10 to 100 parts by mass in 100 parts by mass of the total amount of the polymerizable monomer. From the viewpoint of better adhesiveness with respect to a stereolithographic article, the content of the (meth)acrylamide group-containing polymerizable monomer (a-3) is more preferably 30 to 100 parts by mass and further preferably 50 to 100 parts by mass. In an embodiment that includes the monofunctional (meth)acrylic-based polymerizable monomer (C), the content of the (meth)acrylamide group-containing polymerizable monomer (a-3) is preferably 10 to 98 parts by mass, more preferably 30 to 90 parts by mass, and further preferably 50 to 80 parts by mass in 100 parts by mass of the total amount of the polymerizable monomer.

In a certain embodiment (e.g., an embodiment that does not contain the monofunctional (meth)acrylic-based polymerizable monomer (C)), the content of the (meth)acrylamide group-containing polymerizable monomer (a-3) in the stereolithographic article adhesive composition is preferably 40 to 99.9 mass% in the total amount of the stereolithographic article adhesive composition. From the viewpoint of better adhesiveness with respect to a stereolithographic article, the content of the (meth)acrylamide group-containing polymerizable monomer (a-3) is more preferably 55 to 99.5 mass% and further preferably 60 to 99 mass%. In another certain embodiment (e.g., an embodiment that includes the monofunctional (meth)acrylic-based polymerizable monomer (C)), the content of the (meth)acrylamide group-containing polymerizable monomer (a-3) in the stereolithographic article adhesive composition is preferably 40 to 98 mass% in the total amount of the stereolithographic article adhesive composition. From the viewpoint of better adhesiveness with respect to a stereolithographic article, the content of the (meth)acrylamide group-containing polymerizable monomer (a-3) is more preferably 45 to 90 mass% and further preferably 55 to 85 mass%.

Examples of a multifunctional (meth)acrylate-based polymerizable monomer (a-4) other than the urethanated (meth)acrylic acid ester compound (a-1), the aromatic compound-based (meth)acrylic acid ester compound (a-2) containing no urethane bond, and the (meth)acrylamide group-containing polymerizable monomer (a-3) (hereinafter, also referred to as other multifunctional (meth)acrylate-based polymerizable monomer (a-4)) in the stereolithographic article adhesive composition of the present invention include aliphatic compound-based bifunctional (meth)acrylate-based polymerizable monomers and trifunctional or higher (meth)acrylate-based polymerizable monomers. Examples of the aliphatic compound-based bifunctional (meth)acrylate-based polymerizable monomers include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, and 1,2-bis(3-methacryloyloxy-2-hydroxypropoxy)ethane. Examples of the trifunctional or higher (meth)acrylate-based polymerizable monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, and dipentaerythritol penta(meth)acrylate. Among them, from the viewpoint of excellent adhesiveness with respect to a stereolithographic article, glycerol di(meth)acrylate and 1,2-bis(3-methacryloyloxy-2-hydroxypropoxy)ethane are preferable. As the other multifunctional (meth)acrylate-based polymerizable monomer (a-4), one of these monomers may be used alone, or two or more of these monomers may be used in combination.

In the case where the stereolithographic article adhesive composition of the present invention includes the other multifunctional (meth)acrylate-based polymerizable monomer (a-4), in an embodiment that does not contain the monofunctional (meth)acrylic-based polymerizable monomer (C), the content of the other multifunctional (meth)acrylate-based polymerizable monomer (a-4) in the stereolithographic article adhesive composition of the present invention is preferably 10 to 100 parts by mass, more preferably 30 to 100 parts by mass, and further preferably 50 to 100 parts by mass in 100 parts by mass of the total amount of the polymerizable monomer. In an embodiment that includes the monofunctional (meth)acrylic-based polymerizable monomer (C), the content of the other multifunctional (meth)acrylate-based polymerizable monomer (a-4) is preferably 10 to 98 parts by mass, more preferably 30 to 90 parts by mass, and further preferably 50 to 80 parts by mass in 100 parts by mass of the total amount of the polymerizable monomer.

In a certain embodiment (e.g., an embodiment that does not contain the monofunctional (meth)acrylic-based polymerizable monomer (C)), the content of the other multifunctional (meth)acrylate-based polymerizable monomer (a-4) in the stereolithographic article adhesive composition is preferably 40 to 99.9 mass% in the total amount of the stereolithographic article adhesive composition. From the viewpoint of better adhesiveness with respect to a stereolithographic article, the content of the other multifunctional (meth)acrylate-based polymerizable monomer (a-4) is more preferably 55 to 99.5 mass% and further preferably 60 to 99 mass%. In another certain embodiment (e.g., an embodiment that includes the monofunctional (meth)acrylic-based polymerizable monomer (C)), the content of the other multifunctional (meth)acrylate-based polymerizable monomer (a-4) in the stereolithographic article adhesive composition is preferably 40 to 98 mass% in the total amount of the stereolithographic article adhesive composition. From the viewpoint of better adhesiveness with respect to a stereolithographic article, the content of the other multifunctional (meth)acrylate-based polymerizable monomer (a-4) is more preferably 45 to 90 mass% and further preferably 55 to 85 mass%.

The total amount (total content) of the multifunctional (meth)acrylic-based polymerizable monomer (A) in the stereolithographic article adhesive composition of the present invention is preferably 10 to 100 parts by mass in 100 parts by mass of the total amount of the polymerizable monomer. From the viewpoint of better adhesiveness with respect to a stereolithographic article, the total content of the multifunctional (meth)acrylic-based polymerizable monomer (A) is more preferably 30 to 90 parts by mass and further preferably 50 to 80 parts by mass. Also, in a certain embodiment (e.g., an embodiment that does not contain the monofunctional (meth)acrylic-based polymerizable monomer (C)), the total content of the multifunctional (meth)acrylic-based polymerizable monomer (A) in the stereolithographic article adhesive composition is preferably 40 to 99.9 mass% in the total amount of the stereolithographic article adhesive composition. From the viewpoint of better adhesiveness with respect to a stereolithographic article, the total content of the multifunctional (meth)acrylic-based polymerizable monomer (A) is more preferably 55 to 99.5 mass% and further preferably 60 to 99 mass%. In another certain embodiment (e.g., an embodiment that includes the monofunctional (meth)acrylic-based polymerizable monomer (C)), the total content of the multifunctional (meth)acrylic-based polymerizable monomer (A) in the stereolithographic article adhesive composition is preferably 40 to 98 mass% in the total amount of the stereolithographic article adhesive composition. From the viewpoint of better adhesiveness with respect to a stereolithographic article, the total content of the multifunctional (meth)acrylic-based polymerizable monomer (A) is more preferably 45 to 90 mass% and further preferably 55 to 85 mass%.

### [Photopolymerization initiator (B)]

The stereolithographic article adhesive composition of the present invention needs to contain the photopolymerization initiator (B), since high curability is important to achieve excellent adhesiveness with respect to a stereolithographic article. The photopolymerization initiator (B) used in the present invention can be selected from among polymerization initiators used in general industry, and among them, photopolymerization initiators used in dental applications are preferable.

Examples of the photopolymerization initiator (B) include (bis)acylphosphine oxides, thioxanthones or quaternary ammonium salts thereof, ketals, α-diketones, coumarins, anthraquinones, benzoin alkyl ether compounds, and α-amino ketone-based compounds. As the photopolymerization initiator (B), one of these photopolymerization initiators may be used alone, or two or more of these photopolymerization initiators may be used in combination.

Among these photopolymerization initiators (B), at least one selected from the group consisting of (bis)acylphosphine oxides and α-diketones is preferably used. Accordingly, a stereolithographic article adhesive composition that has excellent photocurability in the ultraviolet and visible light ranges and that exhibits sufficient photocurability even when any light source out of a laser, a halogen lamp, a light-emitting diode (LED), and a xenon lamp is used.

Examples of acylphosphine oxides among the (bis)acylphosphine oxides include 2,4,6-trimethylbenzoyl diphenylphosphine oxide, 2,6-dimethoxybenzoyl diphenylphosphine oxide, 2,6-dichlorobenzoyl diphenylphosphine oxide, 2,4,6-trimethylbenzoyl methoxyphenylphosphine oxide, 2,4,6-trimethylbenzoyl ethoxyphenylphosphine oxide, 2,3,5,6-tetramethylbenzoyl diphenylphosphine oxide, benzoyl di(2,6-dimethylphenyl)phosphonate, a 2,4,6-trimethylbenzoyl phenylphosphine oxide sodium salt, a 2,4,6-trimethylbenzoyl phenylphosphine oxide potassium salt, and an ammonium salt of 2,4,6-trimethylbenzoyl diphenylphosphine oxide. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphineoxide. Furthermore, examples thereof include the compounds described in JP 2000-159621 A.

Among these (bis)acylphosphine oxides, 2,4,6-trimethylbenzoyl diphenylphosphine oxide, 2,4,6-trimethylbenzoyl methoxyphenylphosphine oxide, bis(2,4,6-trimethylbenzoyl)phenylphosphine oxide, and 2,4,6-trimethylbenzoyl phenylphosphine oxide sodium salt are particularly preferably used as the photopolymerization initiator (B).

Examples of the α-diketones include diacetyl, benzyl, camphorquinone, 2,3-pentadione, 2,3-octadione, 9,10-phenanthrenequinone, 4,4'-oxybenzyl, and acenaphthenequinone. Among them, camphorquinone is suitable when a light source in the visible light range is used.

The content of the photopolymerization initiator (B) in the stereolithographic article adhesive composition of the present invention is not particularly limited. From the viewpoint of curability of the obtained stereolithographic article adhesive composition, etc., the content of the photopolymerization initiator (B) is preferably 0.01 to 20 parts by mass with respect to 100 parts by mass of the total amount of the polymerizable monomer. When the content of the photopolymerization initiator (B) is less than 0.01 parts by mass, polymerization may not proceed sufficiently and thus a molded article may not be obtained. The content of the photopolymerization initiator (B) is more preferably 0.05 parts by mass or more, further preferably 0.1 parts by mass or more, and particularly preferably 0.5 parts by mass or more with respect to 100 parts by mass of the total amount. On the other hand, when the content of the photopolymerization initiator (B) exceeds 20 parts by mass with respect to 100 parts by mass of the total amount, if the solubility of the polymerization initiator itself is low, the polymerization initiator may precipitate from the stereolithographic article adhesive composition. The content of the photopolymerization initiator (B) is more preferably 15 parts by mass or less, further preferably 10 parts by mass or less, and particularly preferably 5 parts by mass or less with respect to 100 parts by mass of the total amount.

### [Monofunctional (meth)acrylic-based polymerizable monomer (C)]

The stereolithographic article adhesive composition of the present invention may contain the monofunctional (meth)acrylic-based polymerizable monomer (C). In the stereolithographic article adhesive composition of the present invention, the monofunctional (meth)acrylic-based polymerizable monomer (C) can decrease the viscosity of the stereolithographic article adhesive composition and impart toughness to lined and restored surfaces, thereby further improving adhesiveness. The lined and restored surfaces can be peeled by an excessive stress load, and there are two main mechanisms including the case where the bond between a stereolithographic article and a layer composed of a cured product of the stereolithographic article adhesive composition breaks and the case where the cured product layer composed of the stereolithographic article adhesive composition fractures. By including the monofunctional (meth)acrylic-based polymerizable monomer (C) and imparting toughness, the cured product layer composed of the stereolithographic article adhesive composition is less likely to fracture, and thus adhesiveness can be improved.

Examples of the monofunctional (meth)acrylic-based polymerizable monomer (C) include aromatic ring-containing (meth)acrylate-based polymerizable monomers, alicyclic (meth)acrylate-based polymerizable monomers, nitrogen atom-containing cyclic (meth)acrylate-based polymerizable monomers, chain (meth)acrylate-based polymerizable monomers, cyclic (meth)acrylamide compounds, and chain (meth)acrylamide compounds. As the monofunctional (meth)acrylic-based polymerizable monomer (C), one of these polymerizable monomers may be used alone, or two or more of these polymerizable monomers may be used in combination. From the viewpoint of being less likely to perceive unpleasant odors in handling, the atmospheric-equivalent boiling point of the monofunctional (meth)acrylic-based polymerizable monomer (C) is preferably 250°C or higher. Thus, aromatic ring-containing (meth)acrylate-based polymerizable monomers, alicyclic (meth)acrylate-based polymerizable monomers, nitrogen atom-containing cyclic (meth)acrylate-based polymerizable monomers, and cyclic (meth)acrylamide compounds that tend to have a higher boiling point and from which odors are less likely to be perceived, are preferable. Among them, from the viewpoint of curability and adhesiveness, aromatic ring-containing (meth)acrylate-based polymerizable monomers are more preferable. Aromatic ring-containing (meth)acrylate-based polymerizable monomers, alicyclic (meth)acrylate-based polymerizable monomers, nitrogen atom-containing cyclic (meth)acrylate-based polymerizable monomers, and cyclic (meth)acrylamide compounds tend to have a higher atmospheric-equivalent boiling point and lower odor than chain (meth)acrylate-based polymerizable monomers, when the molecular weights thereof are the same. This is presumably due to π-electron interactions and the effect due to a weak polarity being imparted by fixing the conformation. The atmospheric boiling point in the present invention is a value measured by atmospheric distillation, and for a compound whose atmospheric boiling point cannot be observed, an atmospheric boiling point obtained by conversion from a reduced-pressure boiling point, which is a value measured by reduced-pressure distillation, using a boiling point conversion table (Science of Petroleum, Vol. II. p. 1281(1938)) is used. Furthermore, from the viewpoint of adhesiveness in water, the monofunctional (meth)acrylic-based polymerizable monomer (C) preferably contains no urethane bond, no hydroxyl group, no amino group, and no acidic group. As a certain embodiment, a stereolithographic article adhesive composition including no monofunctional (meth)acrylic-based polymerizable monomer that contains a urethane bond, a hydroxyl group, an amino group, and/or an acidic group is exemplified.

Examples of the aromatic ring-containing (meth)acrylate-based polymerizable monomers include (meth)acrylate-based polymerizable monomers having two aromatic rings such as o-phenylphenol (meth)acrylate, m-phenylphenol (meth)acrylate, p-phenylphenol (meth)acrylate, methoxylated-o-phenylphenol (meth)acrylate, methoxylated-m-phenylphenol (meth)acrylate, methoxylated-p-phenylphenol (meth)acrylate, ethoxylated-o-phenylphenol (meth)acrylate, ethoxylated-m-phenylphenol (meth)acrylate, ethoxylated-p-phenylphenol (meth)acrylate, propoxylated-o-phenylphenol (meth)acrylate, propoxylated-m-phenylphenol (meth)acrylate, propoxylated-p-phenylphenol (meth)acrylate, butoxylated-o-phenylphenol (meth)acrylate, butoxylated-m-phenylphenol (meth)acrylate, butoxylated-p-phenylphenol (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 4-(o-phenoxyphenyl)butyl (meth)acrylate, 4-(m-phenoxyphenyl)butyl (meth)acrylate, 4-(p-phenoxyphenyl)butyl (meth)acrylate, 4-biphenylyl (meth)acrylate, benzhydrol (meth)acrylate, and cumylphenol (meth)acrylate; and (meth)acrylate-based polymerizable monomers having a condensed ring such as 1-naphthyl (meth)acrylate, 2-naphthyl (meth)acrylate, anthryl (meth)acrylate, fluorenyl (meth)acrylate, and fluorenylmethyl (meth)acrylate.

Examples of the alicyclic (meth)acrylate-based polymerizable monomers include 2-(1-adamantyl)propyl (meth)acrylate, 2-methyladamantyl-2-yl (meth)acrylate, 2-ethyladamantyl-2-yl (meth)acrylate, 2-n-propyladamantyl-2-yl (meth)acrylate, 2-isopropyladamantyl-2-yl (meth)acrylate, 1-(adamantan-1-yl)-1-methylethyl (meth)acrylate, 1-(adamantan-1-yl)-1-ethylethyl (meth)acrylate, 1-(adamantan-1-yl)-1 -methylpropyl (meth)acrylate, and 1-(adamantan-1-yl)-1-ethylpropyl (meth)acrylate.

Examples of the nitrogen atom-containing cyclic (meth)acrylate-based polymerizable monomers include multifunctional (meth)acrylate compounds containing a nitrogen-containing heterocyclic group such as pentamethylpiperidinyl (meth)acrylate, tetramethylpiperidinyl (meth)acrylate, and 4-(pyrimidin-2-yl)piperazin-1-yl (meth)acrylate.

Examples of the chain (meth)acrylate-based polymerizable monomers include chain hydrocarbon group-containing (meth)acrylate compounds having 6 to 40 carbon atoms such as 2-ethylhexyl (meth)acrylate, decyl (meth)acrylate, undecyl (meth)acrylate, lauryl (meth)acrylate, tridecyl (meth)acrylate, tetradecyl (meth)acrylate, pentadecyl (meth)acrylate, cetyl (meth)acrylate, palmitoleyl (meth)acrylate, heptadecyl (meth)acrylate, oleyl (meth)acrylate, stearyl (meth)acrylate, and isostearyl (meth)acrylate; and chain hydrocarbon group- and hydroxyl group-containing (meth)acrylate compounds having 4 to 40 carbon atoms such as 4-hydroxybutyl (meth)acrylate, 6-hydroxyhexyl (meth)acrylate, 10-hydroxydecyl (meth)acrylate, glycerol mono(meth)acrylate, erythritol mono(meth)acrylate, and isobornylcyclohexyl (meth)acrylate.

Examples of the cyclic (meth)acrylamide compounds include N-(meth)acryloylpyrrolidine, N-(meth)acryloylpiperidine, N-(meth)acryloyl-2-methylpiperidine, and N-(meth)acryloyl-2,2,6,6-tetramethylpiperidine.

Examples of the chain (meth)acrylamide compounds include N,N-dimethyl (meth)acrylamide, N,N-diethyl (meth)acrylamide, N,N-di-n-propyl (meth)acrylamide, N-isopropyl (meth)acrylamide, N,N-di-n-butyl (meth)acrylamide, N,N-di-n-hexyl (meth)acrylamide, N,N-di-n-octyl (meth)acrylamide, N,N-di-2-ethylhexyl (meth)acrylamide, N-(2-hydroxyethyl) (meth)acrylamide, and N,N,N-bis(2-hydroxyethyl) (meth)acrylamide.

As the monofunctional (meth)acrylic-based polymerizable monomer (C), among them, from the viewpoint that the stereolithographic article adhesive composition of the present invention has a low odor, and has excellent adhesiveness by imparting toughness to lined and restored surfaces, o-phenoxybenzyl acrylate, m-phenoxybenzyl acrylate, p-phenoxybenzyl acrylate, 2-(o-phenoxyphenyl)ethyl acrylate, 2-(m-phenoxyphenyl)ethyl acrylate, 2-(p-phenoxyphenyl)ethyl acrylate, o-phenylphenol (meth)acrylate, m-phenylphenol (meth)acrylate, p-phenylphenol (meth)acrylate, methoxylated-o-phenylphenol (meth)acrylate, methoxylated-m-phenylphenol (meth)acrylate, methoxylated-p-phenylphenol (meth)acrylate, ethoxylated-o-phenylphenol (meth)acrylate, ethoxylated-m-phenylphenol (meth)acrylate, ethoxylated-p-phenylphenol (meth)acrylate, fluorenyl (meth)acrylate, fluorenylmethyl (meth)acrylate, and 4-biphenylyl (meth)acrylate are preferable, m-phenoxybenzyl acrylate, o-phenoxybenzyl acrylate, p-phenoxybenzyl acrylate, o-phenylphenol (meth)acrylate, m-phenylphenol (meth)acrylate, p-phenylphenol (meth)acrylate, ethoxylated-o-phenylphenol (meth)acrylate, ethoxylated-m-phenylphenol (meth)acrylate, and ethoxylated-p-phenylphenol (meth)acrylate are more preferable, and m-phenoxybenzyl acrylate and ethoxylated-o-phenylphenol (meth)acrylate are further preferable.

The content of the monofunctional (meth)acrylic-based polymerizable monomer (C) in the stereolithographic article adhesive composition of the present invention is preferably 1 to 60 parts by mass in 100 parts by mass of the total amount of the polymerizable monomer. From the viewpoint of better adhesiveness with respect to a stereolithographic article, the content of the monofunctional (meth)acrylic-based polymerizable monomer (C) is more preferably 5 to 50 parts by mass and further preferably 10 to 40 parts by mass. Also, in an embodiment that includes the monofunctional (meth)acrylic-based polymerizable monomer (C), the content of the monofunctional (meth)acrylic-based polymerizable monomer (C) is preferably 1 to 55 mass% in the total amount of the stereolithographic article adhesive composition. From the viewpoint of better adhesiveness with respect to a stereolithographic article, the content of the monofunctional (meth)acrylic-based polymerizable monomer (C) is more preferably 5 to 50 mass% and further preferably 10 to 40 mass%.

The polymerizable monomer contained in the stereolithographic article adhesive composition of the present invention may be substantially composed of only the multifunctional (meth)acrylic-based polymerizable monomer (A) or only the multifunctional (meth)acrylic-based polymerizable monomer (A) and the monofunctional (meth)acrylic-based polymerizable monomer (C). The polymerizable monomer being substantially composed of only the multifunctional (meth)acrylic-based polymerizable monomer (A) or only the multifunctional (meth)acrylic-based polymerizable monomer (A) and the monofunctional (meth)acrylic-based polymerizable monomer (C) means that the content of other polymerizable monomers other than the multifunctional (meth)acrylic-based polymerizable monomer (A) or the multifunctional (meth)acrylic-based polymerizable monomer (A) and the monofunctional (meth)acrylic-based polymerizable monomer (C) is less than 10.0 parts by mass, preferably less than 5.0 parts by mass, more preferably less than 1.0 part by mass, further preferably less than 0.1 parts by mass, and particularly preferably less than 0.01 parts by mass in 100 parts by mass of the total amount of the polymerizable monomer contained in the stereolithographic article adhesive composition.

The stereolithographic article adhesive composition of the present invention is not particularly limited as long as the stereolithographic article adhesive composition contains the multifunctional (meth)acrylic-based polymerizable monomer (A) and the photopolymerization initiator (B) described above, and, for example, the stereolithographic article adhesive composition may contain other components other than the above. The stereolithographic article adhesive composition of the present invention can be produced according to a known method.

For the purpose of improving photocurability, the stereolithographic article adhesive composition of the present invention may contain a polymerization accelerator without departing from the gist of the present invention. Examples of the polymerization accelerator include ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. Among them, from the viewpoint of imparting excellent curability to the stereolithographic article adhesive composition, at least one selected from the group consisting of ethyl 4-(N,N-dimethylamino)benzoate, n-butoxyethyl 4-(N,N-dimethylamino)benzoate, and 4-(N,N-dimethylamino)benzophenone is preferably used.

To adjust the applied thickness of an adhesive material and to adjust permeability into a stereolithographic article, the stereolithographic article adhesive composition of the present invention may contain an organic solvent without departing from the gist of the present invention. Examples of the organic solvent include ethyl acetate, butyl acetate, methylene chloride, acetone, ethanol, and isopropanol. One of these organic solvents may be used alone, or two or more of these organic solvents may be used in combination.

To adjust paste properties, the stereolithographic article adhesive composition of the present invention may further contain a filler. Examples of the filler include an organic filler, an inorganic filler, and an organic-inorganic composite filler. One of these fillers may be used alone, or two or more of these fillers may be used in combination.

Examples of the material of the organic filler include polymethyl methacrylate, ethyl polymethacrylate, methyl methacrylate-ethyl methacrylate copolymers, crosslinked polymethyl methacrylate, crosslinked polyethyl methacrylate, polyester, polyamide, polycarbonate, polyphenylene ether, polyoxymethylene, polyvinyl chloride, polystyrene, polyethylene, polypropylene, chloroprene rubber, nitrile rubber, ethylene-vinyl acetate copolymers, styrene-butadiene copolymers, acrylonitrile-styrene copolymers, and acrylonitrile-styrene-butadiene copolymers. One of these organic fillers may be used alone, or two or more of these organic fillers may be used in combination. The shape of the organic filler is not particularly limited, and the particle diameter of the filler can be selected and used as appropriate.

Examples of the material of the inorganic filler include quartz, silica, alumina, silica-titania, silica-titania-barium oxide, silica-zirconia, silica-alumina, lanthanum glass, borosilicate glass, soda glass, barium glass, strontium glass, glass ceramic, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, and strontium calcium fluoroaluminosilicate glass. One of these inorganic fillers may be used alone, or two or more of these inorganic fillers may be used in combination. The shape of the organic filler is not particularly limited, and an irregularly-shaped filler, a spherical filler, or the like can be selected and used as appropriate.

Examples of the organic-inorganic composite filler include inorganic fillers dispersed in organic fillers, and organic-inorganic composite fillers in which inorganic fillers are coated with various polymerizable monomers.

For the purpose of modifying flexibility, flowability, etc., a polymer can be added to the stereolithographic article adhesive composition of the present invention without departing from the gist of the present invention. For example, natural rubber, synthetic polyisoprene rubber, liquid polyisoprene rubber and a hydrogenated product thereof, polybutadiene rubber, liquid polybutadiene rubber and a hydrogenated product thereof, styrene-butadiene rubber, chloroprene rubber, ethylene propylene rubber, acrylic rubber, isoprene-isobutylene rubber, acrylonitrile-butadiene rubber, or a styrene elastomer can be added. Specific examples of other polymers that can be added include polystyrene-polyisoprene-polystyrene block copolymers, polystyrene-polybutadiene-polystyrene block copolymers, poly(α-methylstyrene)-polybutadiene-poly(α-methylstyrene) block copolymers, poly(p-methylstyrene)-polybutadiene-poly(α-methylstyrene) block copolymers, and hydrogenated products thereof.

The stereolithographic article adhesive composition of the present invention may contain a softener as necessary. Examples of the softener include petroleum-based softeners such as paraffinic, naphthenic, and aromatic process oils, and vegetable oilbased softeners such as paraffin, peanut oil, and rosin. One of these softeners may be used alone, or two or more of these softeners may be used in combination. The content of the softener is not particularly limited as long as it does not depart from the gist of the present invention. The content of the softener is normally 200 parts by mass or less and preferably 100 parts by mass or less with respect to 100 parts by mass of the total amount of the polymerizable monomer.

For the purpose of suppressing degradation or adjusting photocurability, the stereolithographic article adhesive composition of the present invention can also contain a known stabilizer. Examples of the stabilizer include a polymerization inhibitor, an ultraviolet absorber, and an antioxidant.

Examples of the polymerization inhibitor include hydroquinone, hydroquinone monomethyl ether, dibutylhydroquinone, dibutylhydroquinone monomethyl ether, 4-t-butylcatechol, 2-t-butyl-4,6-dimethylphenol, 2,6-di-t-butylphenol, and 3,5-di-t-butyl-4-hydroxytoluene. The content of the polymerization inhibitor is preferably 0.001 to 5.0 parts by mass with respect to 100 parts by mass of the total amount of the polymerizable monomer.

For the purpose of adjusting a color tone or adjusting paste properties, the stereolithographic article adhesive composition of the present invention can also contain a known additive. Examples of the additive include a pigment, a dye, a thickener, a polymer, a prepolymer (oligomer), an aryl compound (e.g., an aryl iodonium salt), and a silane coupling agent. One of these additives may be used alone, or two or more of these additives may be used in combination. The content of the additive is not particularly limited, and may be less than 10 mass%, may be less than 1 mass%, or may be less than 0.1 mass%. As a certain suitable embodiment, a stereolithographic article adhesive composition that includes the multifunctional (meth)acrylic-based polymerizable monomer (A) and the photopolymerization initiator (B) and that contains no polymer and no oligomer is exemplified.

The stereolithographic article adhesive composition of the present invention has excellent adhesiveness with respect to a stereolithographic article by having the above configuration. In stereolithography, secondary polymerization is normally carried out to polymerize an unreacted polymerizable monomer after shaping, but the stereolithographic article adhesive composition of the present invention is characterized by having excellent adhesiveness even in a state where polymerization has proceeded to a high degree after this secondary polymerization and after the elapse of a long time from the shaping. Therefore, the stereolithographic article adhesive composition of the present invention can be applied to applications where such an advantage can be utilized, and are particularly optimum for lining and restoring denture base materials and mouthpiece-like sleep disorder treatment tools. In the case of lining a denture base material, the stereolithographic article adhesive composition of the present invention can be applied not only as an adhesive material in conventional lining methods, but also as a denture base lining material itself.

Examples of the stereolithographic article for which the stereolithographic article adhesive composition of the present invention is used include denture base materials, dental mouthpieces, and treatment tools for sleep apnea syndrome. The stereolithographic article is not particularly limited, and examples thereof include a stereolithographic article obtained by performing stereolithography on a composition that includes a polymerizable monomer and a photopolymerization initiator and that contains, as the polymerizable monomer, at least one selected from the group consisting of a urethanated (meth)acrylic acid ester compound (a-1) and an aromatic compound-based (meth)acrylic acid ester compound (a-2) containing no urethane bond. As the urethanated (meth)acrylic acid ester compound (a-1) and the aromatic compound-based (meth)acrylic acid ester compound (a-2) containing no urethane bond, those described for the stereolithographic article adhesive composition can be used, and as the photopolymerization initiator, the photopolymerization initiator (B) described for the stereolithographic article adhesive composition can be used. In a certain embodiment, the stereolithographic article is a stereolithographic article obtained by performing stereolithography on a composition that includes a polymerizable monomer and a photopolymerization initiator and the polymerizable monomer includes a urethanated (meth)acrylic acid ester compound (a-1).

In the stereolithographic article adhesive composition of the present invention, to adjust the thickness and adhesiveness of a coating film for lining and restoring a denture base material or a mouthpiece-like sleep disorder treatment tool, the type and content of each component, the multifunctional (meth)acrylic-based polymerizable monomer (A), the photopolymerization initiator (B), the monofunctional (meth)acrylic-based polymerizable monomer (C), and various optional components (polymerization accelerator, organic solvent, filler, polymer, softener, stabilizer, additive, etc.) can be adjusted as necessary.

When using the stereolithographic article adhesive composition of the present invention to line and restore a shaped article obtained by optical three-dimensional shaping, it is preferable to apply the stereolithographic article adhesive composition to a lined surface and a restored surface of the shaped article and then irradiating the stereolithographic article adhesive composition with light. As a certain embodiment, a method in which the above-described stereolithographic article adhesive composition is used to line or restore a three-dimensionally shaped article (stereolithographic article) produced by an optical three-dimensional shaping method is exemplified. Such a method is, for example, a method for lining and restoring a shaped article obtained by optical three-dimensional shaping, which includes a step of applying the stereolithographic article adhesive composition to a lined surface and a restored surface of a stereolithographic article (especially, a denture base material, a dental occlusal splint, and a sleep disorder treatment tool) and then irradiating the stereolithographic article adhesive composition with light. Any of a conventionally known dental light irradiator (α-Light V, manufactured by J. Morita Corp.) and a post-curing device for stereolithography (Otoflash G171) can be used. Among them, in the present invention, it is preferable to use an active energy beam as light energy for curing the stereolithographic article adhesive composition. The "active energy beam" means an energy beam that can cure the stereolithographic article adhesive composition, such as ultraviolet rays, electron beams, X-rays, radiant rays, and high-frequency waves. Examples of a light source of the active energy beam include lighting such as a halogen lamp, a xenon lamp, a metal halide lamp, a LED, a mercury lamp, and a fluorescent lamp, and a halogen lamp and a LED are particularly preferable.

### EXAMPLES

Next, the present invention will be further specifically described by means of Examples, but the present invention is not limited to these Examples, and many changes can be made within the technical concept of the present invention by those having ordinary knowledge in the art.

Each component used in stereolithographic article adhesive compositions according to Examples and Comparative Examples will be described below with abbreviations.

### [Multifunctional (meth)acrylic-based polymerizable monomer (A)]

### [Urethanated (meth)acrylic acid ester compound (a-1)]

UDMA: 2,2,4-trimethylhexamethylenebis(2-carbamoyloxyethyl) dimethacrylate (manufactured by Kyoeisha Chemical Co., Ltd., molecular weight: 471)
U4TH: N,N'-(2,2,4-trimethylhexamethylene)bis[2-(aminocarboxy)propane-1,3-diol] tetramethacrylate (manufactured by Kyoeisha Chemical Co., Ltd., molecular weight: 673)

### [Aromatic compound-based (meth)acrylic acid ester compound (a-2) containing no urethane bond]

Bis-GMA: 2,2-bis[4-(3-methacryloyloxy-2-hydroxypropoxy)phenyl]propane (manufactured by SHIN-NAKAMURA CHEMICAL Co., Ltd.) [(Meth)acrylamide group-containing polymerizable monomer (a-3)]
MAEA: 2-methacryloyloxyethylacrylamide (manufactured by KJ Chemicals Corporation)

### [Other multifunctional (meth)acrylate-based polymerizable monomer (a-4)]

BMHPE: 1,2-bis(3-methacryloyloxy-2-hydroxypropoxy)ethane (manufactured by SHIN-NAKAMURA CHEMICAL Co., Ltd.)
TEGDMA: triethylene glycol dimethacrylate (manufactured by SHIN-NAKAMURA CHEMICAL Co., Ltd.)

### [Photopolymerization initiator (B)]

TPO: 2,4,6-trimethylbenzoyl diphenylphosphine oxide
BAPO: bis(2,4,6-trimethylbenzoyl)phenylphosphine oxide

### [Monofunctional (meth)acrylic-based polymerizable monomer (C)]

POBA: m-phenoxybenzyl acrylate (manufactured by Kyoeisha Chemical Co., Ltd., atmospheric-equivalent boiling point: 300°C or higher)
EPPA: ethoxylated-o-phenylphenol acrylate (manufactured by SHIN-NAKAMURA CHEMICAL Co., Ltd., atmospheric-equivalent boiling point: 300°C or higher)

### [Polymerization inhibitor]

B HT: 3,5-di-t-butyl-4-hydroxytoluene

### <Reference Example 1> [Production of stereolithographic article resin composition 1]

In a 2 L brown wide-mouth polyethylene bottle, 700 g of 2,2,4-trimethylhexamethylenebis(2-carbamoyloxyethyl) dimethacrylate, 300 g of m-phenoxybenzyl acrylate, 30 g of 2,4,6-trimethylbenzoyl diphenylphosphine oxide, and 5.0 g of BHT were put. A mechanical stirrer was inserted into the bottle, the mixture was stirred at 25°C for 48 hours, and complete dissolution thereof was confirmed. The obtained composition was used as a stereolithographic article resin composition 1.

### <Reference Example 2> [Production of stereolithographic article resin composition 2]

In a 2 L brown wide-mouth polyethylene bottle, 600 g of ethoxylated bisphenol A diacrylate (ABE-300, manufactured by SHIN-NAKAMURA CHEMICAL Co., Ltd.), 400 g of m-phenoxybenzyl acrylate, 30 g of 2,4,6-trimethylbenzoyl diphenylphosphine oxide, and 5.0 g of BHT were put. A mechanical stirrer was inserted into the bottle, the mixture was stirred at 25°C for 48 hours, and complete dissolution thereof was confirmed. The obtained composition was used as a stereolithographic article resin composition 2.

### [Examples 1 to 8 and Comparative Examples 1 to 10]

Pastes as stereolithographic article adhesive compositions according to Examples 1 to 8 and Comparative Examples 1 to 10 were prepared by mixing components in amounts shown in Table 1 and Table 2 at ordinary temperature (20°C±15°C, JIS (Japan Industrial Standards) Z 8703: 1983).

### <Odor>

The stereolithographic article adhesive composition according to each Example and Comparative Example and a rebase adhesive material were evaluated for odor by ten panelists (n=1). Among the ten panelists, the case where the number of panelists who perceived an unpleasant odor was less than two was evaluated as "good", the case where the number of panelists who perceived an unpleasant odor was two or more and less than five was evaluated as "fair", and the case where the number of panelists who perceived an unpleasant odor was five or more was evaluated as "poor". If no unpleasant odor is perceived, there is no problem.

### <Adhesive strength>

For the stereolithographic article resin compositions 1 and 2 obtained in Reference Examples, test pieces having a diameter of 20 mm and a height of 15 mm were shaped using a stereolithography machine (DIGITALWAX (registered trademark) 020D, manufactured by DWS), and curing thereof was completed by applying a flash 2,000 times using a light irradiation machine (Otoflash (registered trademark) G171, manufactured by EnvisionTEC). Then, one flat surface of each test piece was further polished in flowing water with #600 silicon carbide paper (manufactured by Nihon Kenshi Co., Ltd.), and then the water on the surface was blown away with a dental air syringe to obtain a three-dimensionally shaped article.

The stereolithographic article adhesive composition of each Example and Comparative Example was applied to the polished surface of the obtained three-dimensionally shaped article using a brush, was spread thinly with a dental air syringe, and was then irradiated with 1000 flashes of light using a light irradiation machine (Otoflash (registered trademark) G171, manufactured by EnvisionTEC). A silicone sheet having a thickness of about 1.0 mm and having a round hole with a diameter of 4 mm was attached to the applied surface of the stereolithographic article adhesive composition to define an adhesive area. Subsequently, a denture base lining material (KURAREBASE (registered trademark), manufactured by Kuraray Noritake Dental Inc.) was charged into the round hole without applying any dedicated adhesive material, and a 1 cm × 1 cm PET film was press-bonded thereto, a 500 g weight was placed via a glass plate having a length of 1 cm, a width of 1 cm, and a thickness of 5.0 mm, and the test piece was allowed to stand for 1 hour as it was to cure the denture base lining material. In Comparative Example 1, after an adhesive material dedicated for KURAREBASE was applied, the solvent was removed with a dental air syringe, and then KURAREBASE was charged without light irradiation. Then, the weight and the PET film were removed, and the test piece was immersed in distilled water. A total of five adhesion test samples were produced, all samples immersed in distilled water were kept in a thermostatic incubator maintained at 37°C for 24 hours, and then a shear adhesion test was conducted. Average values of n=5 are shown in Table 1 and Table 2. In this test, if the adhesive strength is 5 MPa or higher, the sample can be used in clinical practice. If the adhesive strength is 10 MPa or higher, the adhesive strength is as good as that of the conventional method, and if the adhesive strength is 15 MPa or higher, the adhesive strength is an extremely good adhesive strength exceeding that of the conventional method.

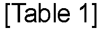

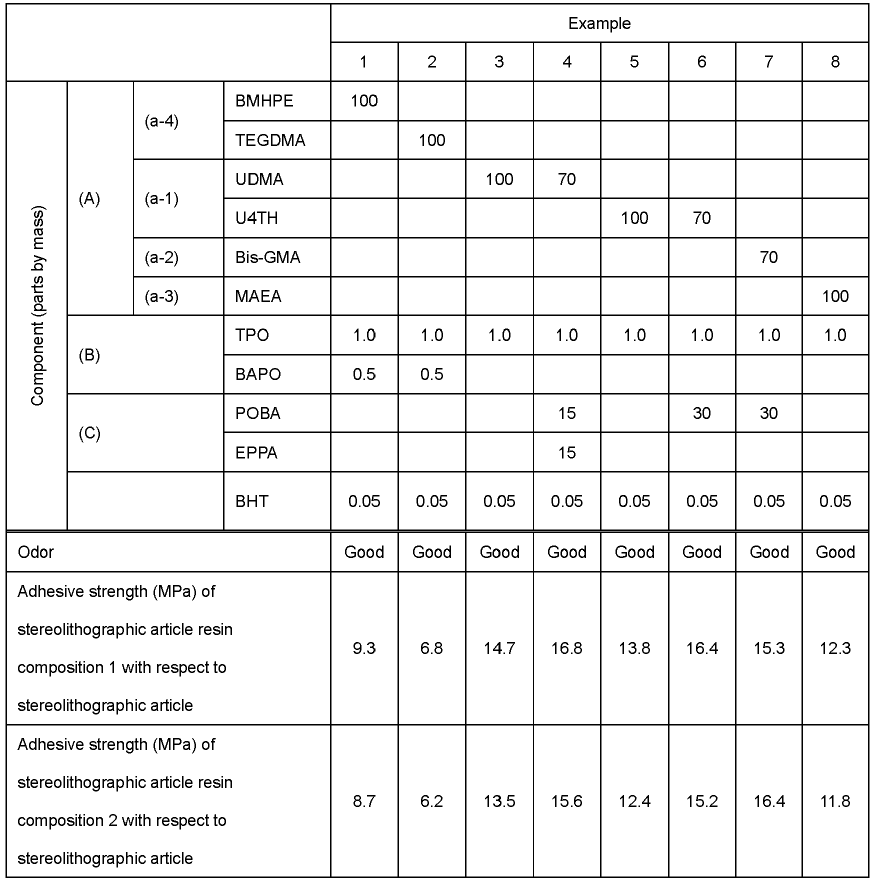

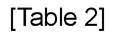

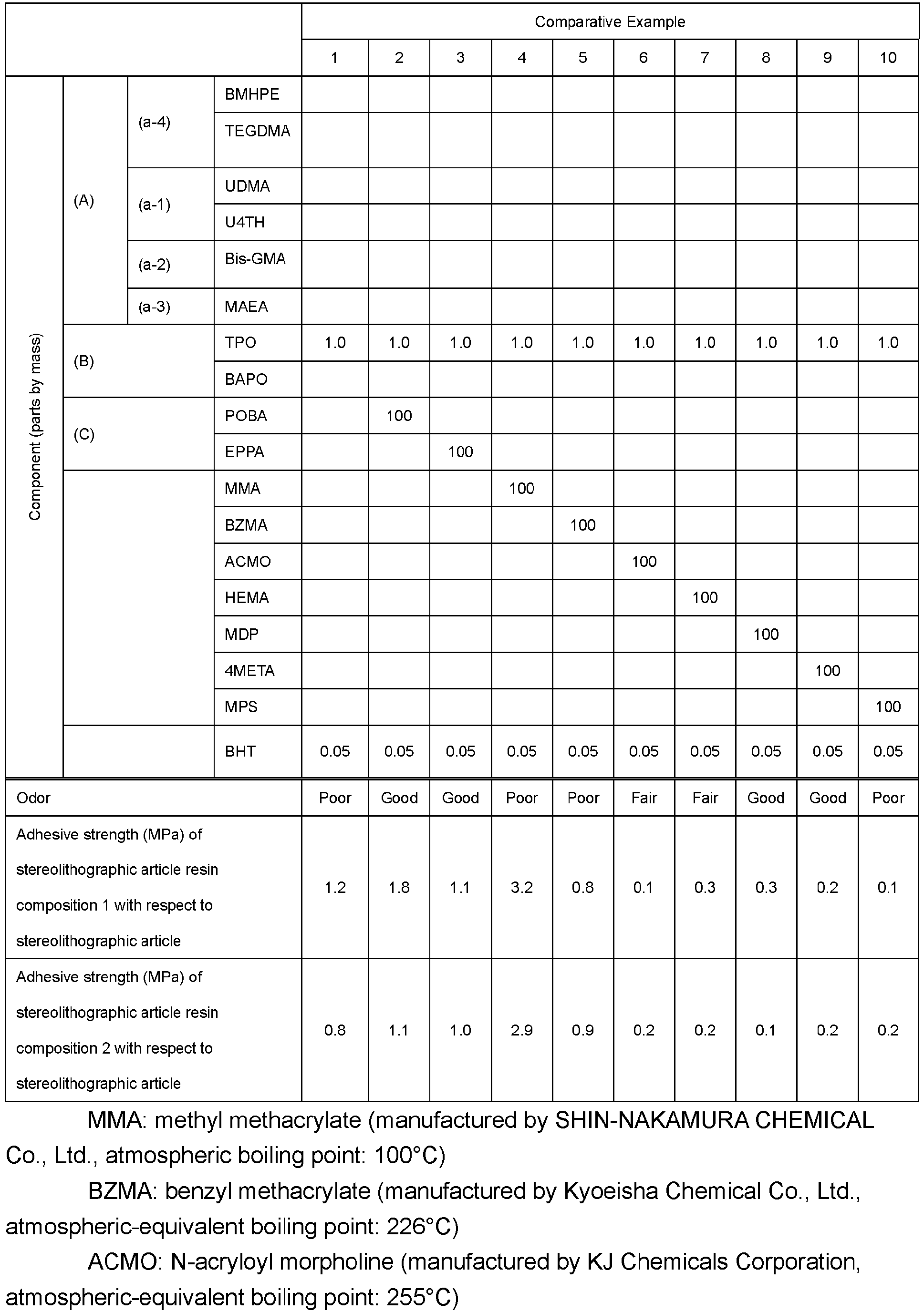

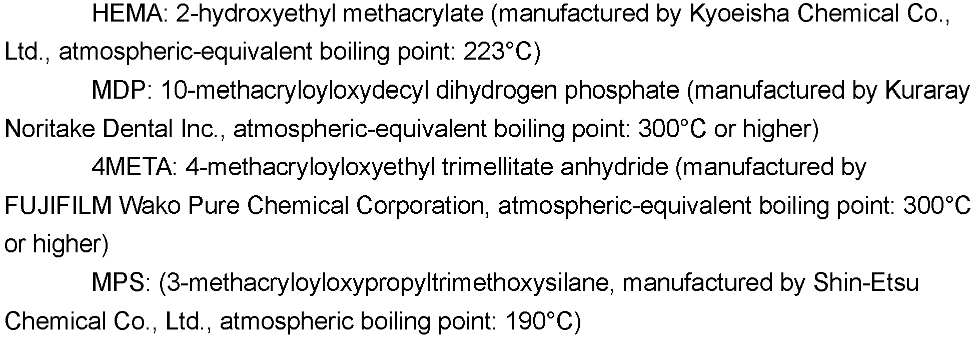

As shown in Table 1 and Table 2, the stereolithographic article adhesive compositions of Examples 1 to 8 had better adhesiveness with respect to an optically, three-dimensionally shaped article than the compositions of Comparative Examples 1 to 10 including no multifunctional (meth)acrylic-based polymerizable monomer (A). In addition, In Examples 1 to 8, a result of having a low odor was obtained. In particular, the odors of the stereolithographic article adhesive compositions according to Examples 1 to 8 were lower and better than those of the compositions of Comparative Examples 1, 4, 5, and 10.

### INDUSTRIAL APPLICABILITY

The stereolithographic article adhesive composition has excellent adhesiveness with respect to a shaped article (three-dimensionally shaped article) produced by stereolithography (optical three-dimensional shaping method) and is capable of restoring or lining a shaped article produced by stereolithography. The stereolithographic article adhesive composition is particularly suitable for lining or restoring denture base materials, dental occlusal splints, and sleep disorder treatment tools, and can be used for a denture base lining material, a denture base lining adhesive material, and a sleep disorder treatment tool restoring material.

## Claims

1. A stereolithographic article adhesive composition comprising a multifunctional (meth)acrylic-based polymerizable monomer (A) and a photopolymerization initiator (B).

2. The stereolithographic article adhesive composition according to claim 1, wherein the multifunctional (meth)acrylic-based polymerizable monomer (A) contains at least one of a urethanated (meth)acrylic acid ester compound (a-1), an aromatic compound-based (meth)acrylic acid ester compound (a-2) containing no urethane bond, and a (meth)acrylamide group-containing polymerizable monomer (a-3).

3. The stereolithographic article adhesive composition according to claim 2, wherein the aromatic compound-based (meth)acrylic acid ester compound (a-2) containing no urethane bond has at least one group selected from the group consisting of a hydroxyl group, a primary amino group, and a secondary amino group.

4. The stereolithographic article adhesive composition according to claim 2, wherein the (meth)acrylamide group-containing polymerizable monomer (a-3) contains at least one bond selected from the group consisting of a primary amide bond and a secondary amide bond.

5. The stereolithographic article adhesive composition according to claim 2, wherein the urethanated (meth)acrylic acid ester compound (a-1) is a compound containing no polymer structure.

6. The stereolithographic article adhesive composition according to claim 5, wherein the urethanated (meth)acrylic acid ester compound (a-1) contains at least one selected from the group consisting of 2,2,4-trimethylhexamethylenebis(2-carbamoyloxyethyl) dimethacrylate and N,N'-(2,2,4-trimethylhexamethylene)bis[2-(aminocarboxy)propane-1,3-diol] tetramethacrylate.

7. The stereolithographic article adhesive composition according to any one of claims 1 to 6, further comprising a monofunctional (meth)acrylic-based polymerizable monomer (C).

8. The stereolithographic article adhesive composition according to claim 7, wherein the monofunctional (meth)acrylic-based polymerizable monomer (C) has an atmospheric-equivalent boiling point of 250°C or higher.

9. The stereolithographic article adhesive composition according to claim 7 or 8, wherein the monofunctional (meth)acrylic-based polymerizable monomer (C) contains no urethane bond, no hydroxyl group, no amino group, and no acidic group.

10. The stereolithographic article adhesive composition according to any one of claims 7 to 9, wherein the monofunctional (meth)acrylic-based polymerizable monomer (C) contains an aromatic ring.

11. The stereolithographic article adhesive composition according to any one of claims 1 to 10, wherein the stereolithographic article is a denture base material, a dental mouthpiece, or a treatment tool for sleep apnea syndrome.

12. A denture base lining material comprising the stereolithographic article adhesive composition according to any one of claims 1 to 10.

13. A denture base lining adhesive material comprising the stereolithographic article adhesive composition according to any one of claims 1 to 10.

14. A sleep disorder treatment tool restoring material comprising a stereolithographic article of the stereolithographic article adhesive composition according to any one of claims 1 to 10.

15. A method for lining or restoring a three-dimensionally shaped article produced by an optical three-dimensional shaping method, using the stereolithographic article adhesive composition according to any one of claims 1 to 11.
